# EUROPEAN PATENT APPLICATION

(11) **EP 4 717 690 A1**
(43) Date of publication of application: **01.04.2026**
(21) Application number: 24202785.2
(22) Date of filing: 26.09.2024
(51) Int. Cl.: C07D 201/00, A61K 9/16, A61K 31/395

(54) **GRANULES COMPRISING 1,4,7,10-TETRAAZACYCLODECANE (CYCLEN)**

(71) Applicant: Midas Pharma GmbH, 55218 Ingelheim (DE)
(72) Inventor: Leone-Stumpf, Danielle, 55268 Nieder-Olm (DE)
(74) Representative: Drescher, Christian

(57) **Abstract**

The present invention relates to granules comprising 1,4,7,10-tetraazacyclodecane (cyclen). Cyclen granules prepared according to the novel granulation process exhibit advantageous properties for transportation.

## Description

The present invention relates to an improved synthesis of 1,4,7,10-tetraazacyclodecane (cyclen).

1,4,7,10-tetraazacyclododecane (cyclen) is a very important intermediate for the synthesis of diagnostics, for example in the preparation of magnetic resonance imaging (MRI), radiological CT, ultrasound imaging and other medical imaging technology contrast agents. As a precursor of macrocyclic chelating agent for the synthesis of metal ions, cyclen and its derivatives form very stable complexes with ions, especially with paramagnetic metal ions such as gadolinium, which can be used in the field of medical diagnosis.

Cyclen may be manufactured according to processes known in the art, e.g. as disclosed in patent application CN 113072514. After crystallization from toluene a white powder of cyclen is obtained.

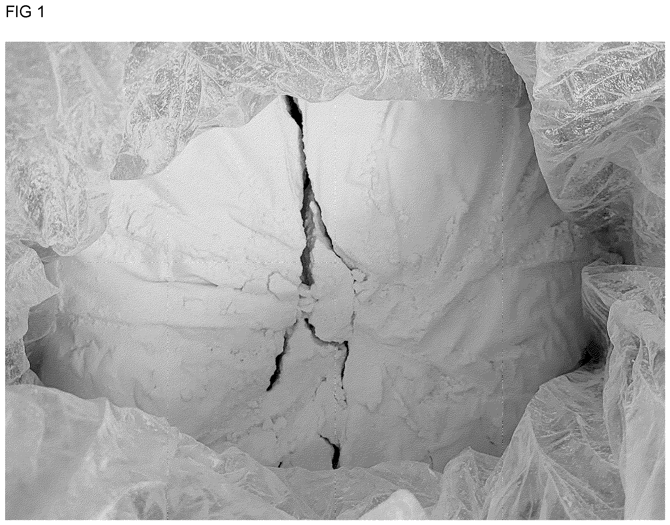

It occurred that cyclen tends to solidify and form a lump upon transportation. Even if packed into sealed PE antistatic bag, thermo-sealed triple-laminated aluminum bag and packed into sealed fiber drums for the sake of protection of the product during transportation, the shipment of cyclen in larger amounts, i.e. in kg-scale for industrial applications, frequently results in an almost total solidification of the complete amount of cyclen, rendering the material practically unsuitable for further processing. An example of a solidified cyclen batch ("lump") is displayed in fig 1.

It has now surprisingly been found that cyclen may be shipped without any tendency to solidify when submitted to a dry granulation process.

Accordingly, the invention relates to a dry granulation process for the manufacture of cyclen granules comprising the following steps:
i. Charging cyclen powder into a granulation apparatus,
ii. Compacting the powder to form a scab,
iii. Breaking up the scab by means of a crushing mill,
iv. Passing the granules through a vibrating screen, and
v. Optionally submitting the granules to an additional sieving step.

Figure 2 shows a schematic drawing of a granulation apparatus.

Figure 3 shows a picture of a batch of cyclen granules that may be obtained by the granulation process according to the present invention.

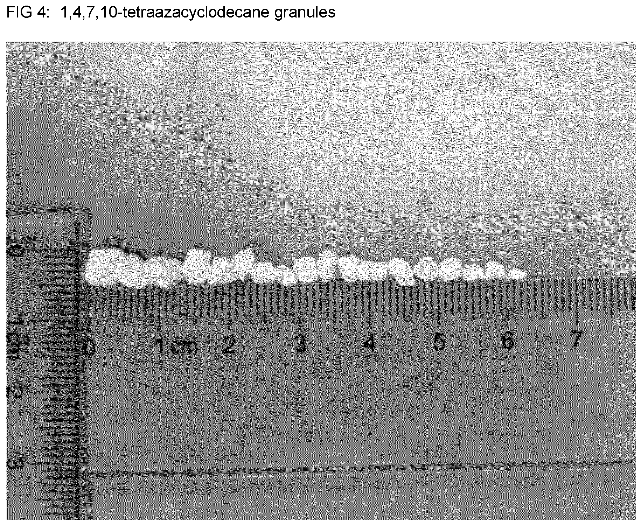

The size of the granules must represent a good compromise between the tendency of solidification and satisfactory dissolution characteristics. It has been found that the tendency to solidify is reduced with increasing size of the granules. On the other hand, it has been observed that the dissolution of the cyclen granules is reduced with increasing size. However, a fast dissolution is advantageous and thus desirable for further processing. The optimal size of the cyclen granules has been determined to be in the range from 1 to 10mm, preferably from 2 to 5 mm. Accordingly, one aspect of the present invention relates to cyclen granules that exhibit an average diameter in the range from 1 to 10mm, preferably between 2 and 5 mm (see Fig. 4).

Surprisingly, the granulation of cyclen was possible without the need to add any excipient. Accordingly, one aspect of the present invention relates to cyclen granules that do not comprise any excipient.

It has been found that the granulation works best when the roll force is kept within the limits of from 4 MPa to 8 MPA. Accordingly, one aspect of the present invention relates to a dry granulation process for the manufacture of cyclen granules wherein the Roll force is in the range of from 4 MPa to 8 MPa, preferable from 5 M Pa to 7 MPa.

It was believed that the tendency to solidify of cyclen powder may be caused by its quite high hygroscopicity. Accordingly, the water uptake of cyclen powder and cyclen granules of the present invention was determined. It turned out that the hygroscopicity of cyclen when reduced when submitted to the granulation process according to the present invention.

For further investigation, a transportation experiment that was intended to simulate typical conditions of shipment was performed. Importantly, cyclen powder and cyclen granules were both packaged with sealed double PE bags and Al-bags and thus put under sealed conditions. Nevertheless, the experiment revealed that the cyclen powder sample solidified and formed a lump whereas the sample with cyclen granules practically remained unchanged.

Without wishing to be bound to theory, the inventors of the present invention trust that the phenomenon of solidification and formation of lumps may be due to certain, yet unknown interparticulate interactions in between the cyclen powder. Accordingly, the tapped and bulk densities of cyclen granules and powder are determined according to standard experimental conditions (USP (616) - *Bulk density of powders*) and compared to each other. Surprisingly, the ration between the tapped and bulk density of cyclen granules is much lower compared to exhibit a ratio of tapped density to bulk density of from 0.9 to 1.1.

### EXAMPLE 1

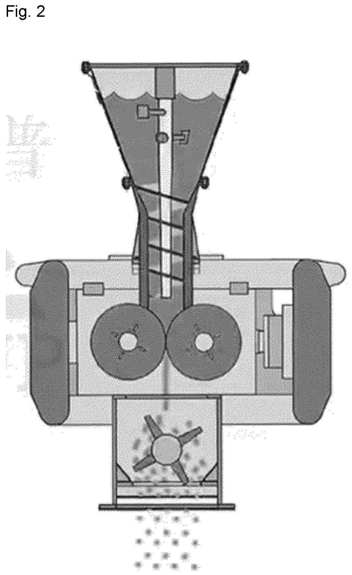

Granules according to the present invention are prepared as follows:
4 kg of cyclen powder are charged into a roller compactor (XIAOLIN GF-150, schematic view see fig. 2). The powder is compacted by counter-rotating rolls to form a scab which is then crushed by means of crushing mills and passed through an oscillating sieve. The compacted granules that are obtained this way are then sieved. The roller compactor is operated at the parameters as displayed in table 1).

### Dry granulation parameter

**Table 1**

| | |
|---|---|
| Charging screw rotation speed | 30 r/min |
| Roller rotation speed | 6.0 r/min |
| Roller diameter | 150mm |
| Roller distance | 1 mm |
| Roll force (pressure) | 6 MPa |
| Roll temperature | 25°C |
| Crushing mill/ vibrating screen | 80 rpm/min / 6 mesh |
| Sieve | 4 mm |

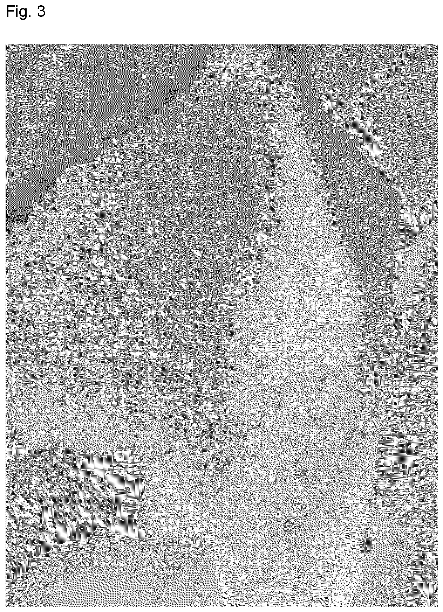

Figure 3 shows a picture of a batch of cyclen granules that was obtained by the abovementioned granulation process.

### EXAMPLE 2 - Characterization and analysis of cyclen granules

The cyclen granules were submitted to characterization and analysis. Results are displayed in table 2 below.

**Table 2**

| **Substance** | **Measured value** | **Specification** |
|---|---|---|
| Unspecified impurities | <0.05% | ≤0.05% |
| Specified impurities | <0.1% | ≤0.1% |
| Total impurities | <0.05% | ≤ 0.5% |
| Water | 0.2% | ≤ 0.5% |
| Assay by titration | 99.6% | 98.0% - 102.0% |

As displayed in table 2, all values such as assay, impurities, etc. fulfil the required specifications.

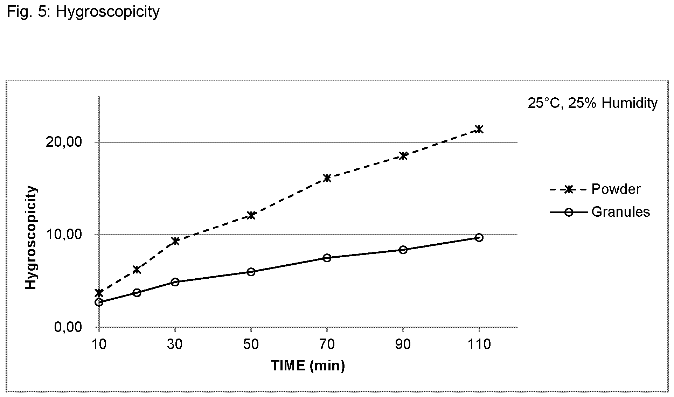

Furthermore, the moisture uptake of the cyclen granules was tested and compared to cyclen powder (25°C / 25% humidity). The results are displayed in table 3 and fig 5.

**Table 3**

| Hygroscopicity °/°° | **10min** | **20min** | **30min** | **50min** | **70min** | **90min** | **110min** |
|---|---|---|---|---|---|---|---|
| **Granules** | 2.70 | 3.72 | 4.86 | 5.96 | 7.48 | 8.35 | 9.67 |
| **Powder** | 3.70 | 6.21 | 9.28 | 12.07 | 16.13 | 18.53 | 21.39 |

As shown in table 3, the hygroscopicity of the cyclen granules is significantly reduced by the application of the dry granulation process.

A comparison of the tapped bulk and untapped ("bulk") densities provide an indirect measure of the interparticulate interactions influencing the properties of a powder. The tapped and bulk densities of cyclen granules and powder are determined according to USP (616) - *Bulk density of powders.* The results are displayed in table 4:

**Table 4**

| | **Bulk density** | **Tapped density** | **Ratio tapped/bulk density** |
|---|---|---|---|
| **Granules** | 0.51g/cm³ | 0.52g/cm³ | 1.02 |
| **Powder** | 0.54 g/cm³ | 0.78 g/cm³ | 1.44 |

As can be deducted from table, the tapped density of the cyclen powder is much higher than the tapped density, indicating that the density of cyclen may tremendously increase upon transportation - which may lead to the observed formation of lump. On the other hand, granulation of cyclen results in the cyclen to be practically inert against the phenomena of transportation.

### EXAMPLE 3 - Transportation simulation experiment

The cyclen were submitted to a transportation simulation experiment. 1 kg samples with cyclen granules and cyclen powder were packaged with double PE bags and Al-bags and put into separated carton drums. A 5 kg weight was put on each of the samples to stress the condition in the drum. The drums were put on a fork lift truck and moved with the truck for 10 days. After that time, the cyclen powder and cyclen granules were submitted to visual and tactile inspection. The granules remained unchanged and easy to handle whereas the powder sample solidified and had transformed into a hard lump which was practically unsuitable for further processing.

## Claims

1. Granules of 1,4,7,10-tetraazacyclodecane.

2. The granules of 1,4,7,10-tetraazacyclodecane according to claim 1 wherein the diameter of the cyclen granules is from 1mm to 10mm, preferably from 2mm to 5 mm.

3. The granules of 1,4,7,10-tetraazacyclodecane according to claim 1 or 2 that do not comprise any additional excipient.

4. The granules of 1,4,7,10-tetraazacyclodecane according to any of the preceding claims wherein the cyclen granules exhibit a ratio of tapped density to bulk density of from 0.9 to 1.1.

5. A process for the manufacture of granules of 1,4,7,10-tetraazacyclodecane according to any of the preceding claims, comprising the steps of:
i. Charging cyclen powder into a granulation apparatus;
ii. Compacting the powder to form a scab;
iii. Breaking up the scab by means of a crushing mill;
iv. Passing the granules through a vibrating screen; and
v. optionally submitting the granules to an additional sieving step.

6. A process for the manufacture of granules of 1,4,7,10-tetraazacyclodecane according to claim 5, wherein no additional excipient is added.

7. A process for the manufacture of granules of 1,4,7,10-tetraazacyclodecane according to claim 5 or 6, **characterized in that** the roll force is in the range of from 4 MPa to 8 MPa
